# EUROPEAN PATENT APPLICATION

(11) **EP 4 779 012 A1**
(43) Date of publication of application: **22.07.2026**
(21) Application number: 24865485.7
(22) Date of filing: 11.09.2024
(51) Int. Cl.: C12M 1/34, C12N 5/077, G01N 27/00, G01N 33/48, G01N 33/483

(54) **DEVICE CAPABLE OF SIMULTANEOUSLY MEASURING MYOCARDIAL-SPECIFIC CONTRACTILE FORCE AND ACTIVITY POTENTIAL**

(30) Priority: 12.09.2023 JP 2023147901
(71) Applicant: Engitissue Design Co., Ltd., Osaka-shi, Osaka 533-0024 (JP); The University of Osaka, Osaka 565-0871 (JP)
(72) Inventor: LIU, Li, Suita-shi, Osaka 565-0871 (JP); LI, Junjun, Suita-shi, Osaka 565-0871 (JP); MIYAGAWA, Shigeru, Suita-shi, Osaka 565-0871 (JP); SAWA, Yoshiki, Tokyo 103-0023 (JP); TAKEDA, Maki, Tokyo 103-0023 (JP)
(74) Representative: Marks & Clerk LLP
(86) International application number: PCT/JP2024/032578
(87) International publication number: WO 2025/057985

(57) **Abstract**

An object is to provide a device for simultaneously measuring electrical signals and contractile force using highly mature myocardial tissue having a three-dimensional, aligned structure similar to that of the myocardial structure in vivo. A solution provided is a device for measuring contractile force of myocardial tissue, including a contraction unit including rod-shaped engineered myocardial tissue with a 3D structure in which cardiomyocytes are aligned in a long-axis direction, and the device is for use in the measurement of contractile force of the contraction unit.

## Description

### Technical Field

The present invention relates to engineered myocardial tissue, use thereof, and a production method thereof.

### Background Art

Conventional toxicity evaluation of pharmaceuticals and drug discovery and development have employed primary cells derived from animals and experimental animals; however, such approaches suffer from various problems, including interspecies differences from human living tissues and lot-to-lot variation. Currently, guidelines require conducting nonclinical studies on cardiotoxicity and QT prolongation of electrocardiograms for drugs across all therapeutic areas. Drugs suspected of inducing lethal arrhythmias due to QT prolongation are forced to be withdrawn from the market, face approval delays, or abandon development. The successful establishment of human iPS cells has created a high demand in industry for applications such as toxicity evaluation and pharmacokinetic evaluation using human iPS cell-derived cardiomyocytes. Although issues with efficiency of differentiation induction and purity of human iPS cell-derived cardiomyocytes have been largely resolved, challenges remain such as low maturity of induced cardiomyocytes, random cell orientation with poor alignment leading to weak contractile force, frequent arrhythmias in response to QT prolongation during drug toxicity evaluation, and large lot-to-lot variation. Thus, there is demand for the development of methods to evaluate drug safety and efficacy using highly mature cardiac tissue that has the same structure as cardiac tissue in vivo.

Conventional methods for evaluating the function of cardiomyocytes commonly include action potential measurement using multielectrodes, and contractile force measurement based on cell motion or electrical impedance. Multi-electrode arrays used for measuring action potentials have the following drawbacks: (1) the adhesion between the multi-electrode chip and cells is weak, causing the cells to aggregate unevenly and detach easily from the electrode chip, and resulting in non-uniform propagation directions of electrical excitation in response to pacing, frequent arrhythmias following drug addition, and the inability to culture cells on multielectrodes for extended periods; (2) the cardiomyocytes on the multi-electrode chip form a random structure, and do not have the native myocardial orientation; and (3) measurement of two-dimensional (2D) cardiomyocytes is possible, but measurement of thick myocardial tissue cannot be performed. Methods such as cell motion imaging and electrical impedance, which are most commonly used to measure the contractile force of cardiomyocytes, are suitable for evaluating contractile force of myocardia by analyzing the beating behavior of 2D cardiomyocytes, but are not applicable to three-dimensional (3D) myocardial tissue having a thick oriented structure.

### Citation List

### Non-patent Literature

NPL 1: T. Hayakawa et al., Journal of Molecular and Cellular Cardiology, 77, 178-191, 2014
NPL 2: F. Qian et al., Lab on a Chip, 17, 1732-1739, 2017
NPL 3: T. Ohys et al., Lab on a Chip, 21, 3899-3909, 2021
NPL 4: J. U. Lind et al., Lab on a Chip, 17, 3692-3703
NPL 5: Yimu Zhao et al., Cell 176, 913-927
NPL 6: I. Mannhardt et al., Stem Cell Reports, 15, 983-998, 2020

### Summary of Invention

### Technical Problem

Current cardiomyocyte function evaluation methods are capable of measuring cardiomyocyte-specific action potentials and contractile force individually. However, the present inventors have required the development of a device and a method for simultaneously measuring both functions under the same environment. Although several methods for simultaneously measuring action potentials and contractile force have been recently reported, all of them assess cardiomyocytes at the two-dimensional level. Accordingly, an object of the present invention is to provide a device that simultaneously measures electrical signals and contractile force using highly mature myocardial tissue having a three-dimensional and oriented structure similar to the myocardial structure in vivo.

### Solution to Problem

The present inventors conducted intensive research to achieve the object, and found that they can offer a device for measuring contractile force of highly mature myocardial tissue having a structure similar to the myocardial structure in vivo by designing a device with a contraction unit containing a 3D-structured, rod-shaped engineered myocardial tissue having cardiomyocytes aligned in the long-axis direction so as to be capable of measuring the contractile force of the contraction unit. The device can be easily designed so as to be capable of simultaneously measuring electrical signals. The present invention was completed by conducting further studies based on the finding, and includes the following aspects.

### Item 1.

A device for measuring contractile force of myocardial tissue, comprising a contraction unit including a rod-shaped engineered myocardial tissue with a 3D structure in which cardiomyocytes are aligned in a long-axis direction,
wherein the device is for use in the measurement of contractile force of the contraction unit.

### Item 2.

The device for measuring contractile force of myocardial tissue according to Item 1, wherein the engineered myocardial tissue is obtained by culturing cardiomyocytes on a surface having a contact angle with water of 95° or greater.

### Item 3.

The device for measuring contractile force of myocardial tissue according to Item 1 or 2, further comprising a marker unit including a non-contractile myocardial tissue,
wherein the contraction unit is coupled to the marker unit so that the contractile force of the contraction unit is measurable according to the degree of displacement of the marker unit.

### Item 4.

The device for measuring contractile force of myocardial tissue according to any one of Items 1 to 3, further comprising an action potential measurement unit including an engineered myocardial tissue with a 3D structure in which cardiomyocytes are aligned,
wherein the action potential measurement unit is for use in the measurement of an action potential with multielectrodes.

### Item 5.

A method for producing a rod-shaped engineered myocardial tissue with a 3D structure in which cardiomyocytes are aligned in a long-axis direction,
the method comprising the step of culturing cardiomyocytes on a surface having a contact angle with water of 95° or greater to obtain the engineered myocardial tissue.

### Item 6.

The method according to Item 5,
wherein
in the culturing step, a region having a surface with a contact angle with water of 95° or greater (A) is joined to two regions each having a surface with a contact angle with water of 85° or less (B), and
cardiomyocytes are cultured in the region (A) and the regions (B) to form the rod-shaped engineered myocardial tissue on a line connecting the two regions (B).

### Advantageous Effects of Invention

The present invention provides a device for measuring contractile force of highly mature myocardial tissue having a structure similar to the myocardial structure in vivo. Such a device can easily be designed so as to be capable of simultaneously measuring electrical signals.

### Brief Description of Drawings

Fig. 1 is a schematic diagram of an example of the device for measuring contractile force.
Fig. 2 shows photographs of an example of the device for measuring contractile force.
Fig. 3 is a schematic diagram showing the measurement principle of the device for measuring contractile force.
Fig. 4 shows an example of measurement results using the device for measuring contractile force.
Fig. 5 shows an example of measurement results using the device for measuring contractile force (the effect of addition of E4031).
Fig. 6 shows an example of measurement results using the device for measuring contractile force (the effect of addition of verapamil).
Fig. 7 (A and B) shows an example of measurement results using the device for measuring contractile force (drug response evaluation using normal and diseased iPS cell-derived cardiomyocytes).
Fig. 8 is a schematic diagram showing an example of applications of the device for measuring contractile force.
Fig. 9 shows photographs illustrating the measurement results of contact angles between various materials with water.
Fig. 10 shows a graph illustrating the measurement results of contact angles between various materials with water.

### Description of Embodiments

### 1. Device for Measuring Contractile Force of Myocardial Tissue

The device for measuring contractile force of myocardial tissue of the present invention comprises a contraction unit including a rod-shaped engineered myocardial tissue with a 3D structure in which cardiomyocytes are aligned in the long-axis direction, and the device is used for measuring the contractile force of the contraction unit.

In the device for measuring contractile force of myocardial tissue of the present invention, the contraction unit includes a rod-shaped engineered myocardial tissue that has a 3D structure and that has cardiomyocytes aligned in the long-axis direction. The contractile force of this contraction unit can be measured. This makes it possible to measure the contractile force of highly mature myocardial tissue having a structure similar to the myocardial structure in vivo. Additionally, as described below, such a device can be easily designed so as to be able to measure electrical signals simultaneously.

The engineered myocardial tissue can be obtained by culturing cardiomyocytes on a surface having a contact angle with water of 95° or greater. Preferably, in the culturing step, a region having a surface with a contact angle with water of 95° or greater (A) is joined to two regions capable of non-alignment culture (B), and culturing cardiomyocytes in the region (A) and the regions (B) enables the formation of the rod-shaped engineered myocardial tissue on a line connecting the two regions (B) .

For example, an elongated strip having a surface with a contact angle with water of 95° or greater (a) is prepared, and both ends of the strip in the long-axis direction are each joined to a substrate having a surface with a contact angle with water of 85° or less (b). Culturing cardiomyocytes in suspension using a liquid medium on them (i.e., (a) and (b)) is likely allow the cardiomyocytes to first gather on the substrates (b) at both ends and form a cardiomyocyte population in situ. On the other hand, cardiomyocytes weakly adhered onto the elongated strip having a surface with a contact angle with water of 95° or greater (a); thus, cardiomyocytes are less likely to accumulate compared to the substrates (b) present at both ends. However, the present inventors found that continuing culture allows the cardiomyocytes to gradually accumulate in the central portion of the elongated strip (a) along the long-axis direction, and to form a rod-shaped engineered myocardial tissue oriented in the long-axis direction in a manner bridging the cardiomyocyte populations at both ends. The inventors confirmed that this rod-shaped engineered myocardial tissue has a 3D structure, and that the cardiomyocytes are aligned in the long-axis direction, thereby constituting a highly mature myocardial tissue having a structure similar to the myocardial structure in vivo. This principle can be used to obtain engineered myocardial tissue of a desired shape regardless of the shape of the surface with a contact angle with water of 95° or greater (regardless of an elongated strip). This principle can also be used to obtain engineered myocardial tissue regardless of whether the substrates (b) are present.

In the initial stage of culture, it is preferable to subject the surface of the elongated strip (a) to plasma treatment in order to facilitate accumulation of cardiomyocytes on the elongated strip (a). In this case, the contact angle of the surface with water temporarily decreases to 85° or less, and therefore cardiomyocytes easily accumulate on the entire surface of the elongated strip (a) immediately after the start of culture. However, as culture continues, the effect of the plasma treatment gradually diminishes, and the contact angle of the surface with water gradually returns to the value before the plasma treatment. Thus, as described above, cardiomyocytes gradually accumulate at the central portion of the elongated strip (a).

From the standpoint of efficiently obtaining engineered myocardial tissue, the surface with a contact angle with water of 95° or greater preferably has a contact angle with water of 100° or greater, and more preferably 105° or greater. From the standpoint of efficiently obtaining engineered myocardial tissue, the surface preferably has a contact angle with water of 120° or less, more preferably 115° or less, and even more preferably 110° or less.

The surface with a contact angle with water of 95° or greater is not particularly limited in terms of material as long as the contact angle with water is within the above range. Examples of materials include silicone (silicon resin), with polydimethylsiloxane (PDMS), silicone rubber, and the like being particularly preferred.

From the standpoint of efficiently obtaining engineered myocardial tissue, the substrate (b) having a surface with a contact angle with water of 85° or less preferably has a contact angle with water of 80° or less, more preferably 75° or less, and even more preferably 70° or less. From the standpoint of efficiently obtaining engineered myocardial tissue, the substrate (b) preferably has a contact angle with water of 50° or greater, and more preferably 60° or greater.

The substrate (b) has a contact angle with water as described above, and can be widely selected from materials known to be suitable for cell culture. Examples include polyimines, polyethylene terephthalate, polyethyleneimine, polystyrene, and glass. Alternatively, the aligned fiber described below may be used as the substrate (b). In this case, the engineered myocardial tissue formed in this region can be aligned.

In the present invention, the contact angle with water is determined by irradiating a droplet on a surface to be measured with light, capturing an image of the droplet with a camera from the opposite side, and perform computing based on image analysis.

The rod-shaped engineered myocardial tissue highly expresses β-MHC, which is a maturation marker. In the present invention, whether myocardial tissue is highly expressing β-MHC can be confirmed by immunohistochemical staining.

From the standpoint of convenience in measuring contractile force, the rod-shaped engineered myocardial tissue has a width of preferably 0.03 to 30 mm, more preferably 0.1 to 10 mm, and even more preferably 0.3 to 3 mm, in the culture surface direction. In a similar regard, the rod-shaped engineered myocardial tissue has a width of preferably 0.003 to 30 mm, more preferably 0.03 to 3 mm, and even more preferably 0.1 to 1 mm, in a direction perpendicular to the culture surface.

The type of cardiomyocytes can be appropriately selected according to the purpose of measurement. For example, for evaluating cardiotoxicity of drugs or the like, cardiomyocytes derived from normal human iPS cells can be used. For drug discovery and development, cardiac disease-derived iPS cell-derived cardiomyocytes can also be used, as necessary.

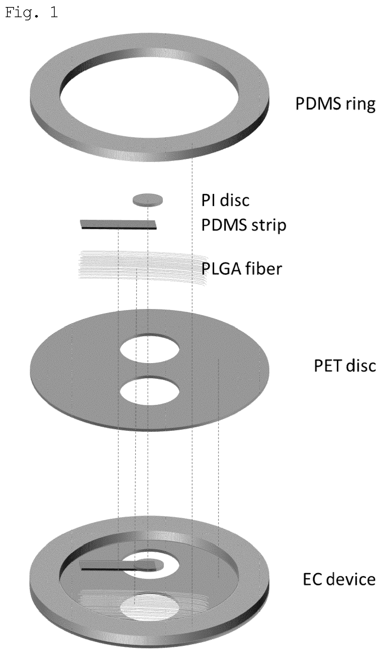

An example of specific configuration of the device for measuring contractile force of myocardial tissue of the present invention is one that includes, as shown in Fig. 1, a substrate at the bottom with the peripheral region of the contraction unit being hollow. This configuration offers the advantage that the contraction unit is more prone to floating, thereby facilitating the measurement of the contractile force. Additionally, as shown in Fig. 1, a configuration in which the periphery is surrounded by a partition member can be optionally used.

In the above case, the material of the member having a hollow section is not particularly limited, and examples include polyethylene terephthalate, polyethyleneimine, and polystyrene.

In the above case, the purpose of the partition member is to prevent leakage of the liquid medium to the outside, and the shape is not particularly limited as long as that purpose is achieved. For example, the partition member can be a ring-shaped member. The material of the partition member is not particularly limited, and examples include polydimethylsiloxane (PDMS), polystyrene, silicone rubber, and glass.

The device for measuring contractile force of myocardial tissue of the present invention may further comprise a marker unit including non-contractile myocardial tissue, wherein the contraction unit may be coupled to the marker unit so that the contractile force of the contraction unit is measurable according to the degree of displacement of the marker unit.

Specifically, the contraction unit and the marker unit are allowed to suspend in a liquid medium. During myocardial pulsation, the amount of displacement of the floating marker unit is detected by image recognition technology. The magnitude of the motion vector and the temporal change are recorded and reconverted into force by calculation to measure the contractile force.

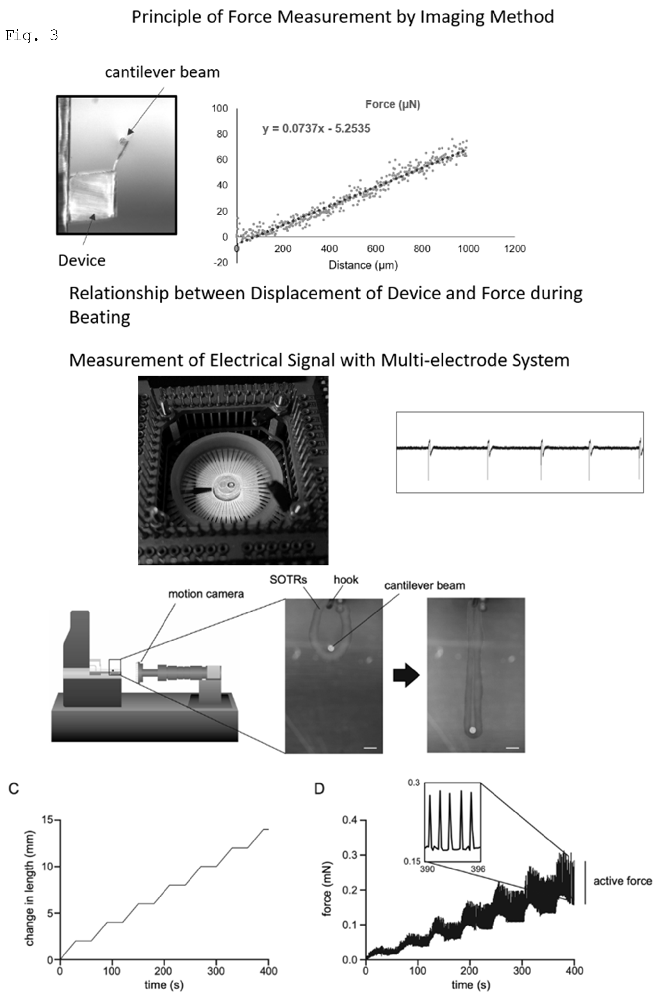

The measurement of contractile force based on the amount of displacement of the marker unit is performed, more specifically, for example, as follows. As shown in Fig. 3, a device for contracting myocardial tissue, MicroTester (CellScale), is used. Since the sensor in contact with tissue moves in sync with myocardial pulsation, the amount of displacement of the sensor is recorded on video. Thereafter, displacement curves of the sensor at different times are obtained using the MUSCLEMOTION plug-in of the free software ImageJ. Finally, this information is used and converted into intracellular forces that the sensor receives at different times.

In the above example, the marker unit may include myocardial tissue formed in the regions having a surface with a contact angle with water of 85° or less (B).

In the present invention, the phrase "non-contractile myocardial tissue" means that myocardial tissue has contractility lower than that of the rod-shaped engineered myocardial tissue of the contraction unit, and does not necessarily mean that myocardial tissue has no contractility. This is because the degree of alignment of the cardiomyocytes is lower than that in the rod-shaped engineered myocardial tissue of the contraction unit.

The device for measuring contractile force of myocardial tissue of the present invention may further comprise an action potential measurement unit including a 3D-structured engineered myocardial tissue in which cardiomyocytes are aligned, and the action potential measurement unit may be for use in the measurement of an action potential with multielectrodes. This enables the provision of a device that simultaneously measures electrical signals and contractile force.

Specifically, an existing multi-electrode system for measuring electrical signals can be applied. A portion of the device can be adhered to the multi-electrode chip to measure action potentials.

The method for obtaining a 3D-structured, engineered myocardial tissue, in which cardiomyocytes are aligned, of the action potential measurement unit is not particularly limited. For example, as previously reported by the present inventors, such myocardial tissue can be obtained by culturing cardiomyocytes using aligned fibers as a cell scaffold (J. Li et al., 2017 Stem Cell Reports, 9, 1-14, 2017). The aligned fibers for use may be, for example, those obtained from a polymer (starting material) by electrospinning. The polymer may be any polymer that does not adversely affect the proliferation and physiological activity of cardiomyocytes, and can be selected widely according to the intended use. Examples include poly(lactic-co-glycolic) acid (PLGA), polylactic acid (PLA), polystyrene (PS), polyethylene terephthalate (PET), gelatin, and collagen.

The diameter of the aligned fibers is not particularly limited; for example, the aligned fibers for use have a diameter of 0.1 to 10 µm, preferably 1 to 8 µm, and more preferably 3 to 5 µm.

When a multi-electrode system is used to measure action potentials with a portion of engineered myocardial tissue adhered to a multi-electrode chip, the aligned fibers need to be formed into a sheet with an area sufficient to obtain the engineered myocardial tissue with the required area.

The number (density) of aligned fibers per millimeter of width in the short-axis direction (the direction perpendicular to the alignment direction) can vary depending on the diameter of fibers used. The density is 10 fibers/mm or greater, preferably 30 to 15,000 fibers/mm, and more preferably 50 to 13,000 fibers/mm.

For example, a device containing one set of the contraction unit and the action potential measurement unit is made to have a size that can be accommodated within a single well of a multi-well microplate (e.g., microplates with 24 wells, 48 wells, or 96 wells are commonly used). This device is taken as one unit, and multiple units of this device are connected so that individual units are accommodated in respective wells of a multi-well microplate. This enables simultaneous measurement of multiple-unit devices by using a commonly used multi-well microplate.

### 2. Method for Producing Rod-shaped Engineered Myocardial Tissue

The method for producing a rod-shaped engineered myocardial tissue with a 3D structure in which cardiomyocytes are aligned in a long-axis direction of the present invention comprises the step of culturing cardiomyocytes on a surface having a contact angle with water of 95° or greater to obtain the engineered myocardial tissue.

In the above step, the culture method is not particularly limited and can be appropriately selected from methods known as culture methods for cardiomyocytes. Culture may be performed in a stationary state, or may be performed with shaking.

In the step above, a region having a surface with a contact angle with water of 95° or greater (A) may be joined to two regions each having a surface with a contact angle with water of 85° or less (B), and cardiomyocytes may be cultured in the region (A) and the regions (B) to form the rod-shaped engineered myocardial tissue on a line connecting the two regions (B). This step enables the production of the device for measuring contractile force of myocardial tissue comprising a marker unit including non-contractile myocardial tissue described above.

### Examples

The present invention is described below with reference to Examples. However, the present invention is not limited to these Examples.

### (1) Fiber Sheet Processing

PLGA (75/25; Sigma-Aldrich, USA) was mixed with hexafluoro-2-propanol (HFIP, Wako Pure Chemical Industries, Tokyo, Japan) in a centrifuge tube (1.2 g: 3 mL, w/v), and fibers were synthesized using this mixture with an automatic fiber production apparatus (NF-103, MECC, Fukuoka, Japan). Specifically, a 3 mL syringe was filled with the solution prepared by mixing and connected to the anode of a high-voltage power source (10 kV) to which a needle with an inner diameter of 0.6 mm was connected. An aluminum foil sheet was placed on the surface of a drum. The drum was rotated at a speed of 1000 rpm, and PLGA fibers ejected from the needle were collected on the aluminum foil sheet. The distance between the needle tip and the drum was maintained at 15 cm, and the spinning process was set to 60 minutes. Thereafter, the fiber sheet collected on the aluminum foil was transferred to a device. The fibers were evaluated by observation with a scanning electron microscope.

### (2) Preparation of Device

PDMS (SYLGARD 184; Dow Corning, Midland, MI, USA) was poured into a culture plate (Nunc Cell Culture Plate, Thermo fisher Scientific) and cured at 80°C overnight to prepare a PDMS sheet with a thickness of 1 mm. PDMS was poured onto a silicon wafer rotating at a speed of 1500 rpm with a spin coater and cured overnight at 80°C to prepare a PDMS sheet with a thickness of 100 µm. The PDMS sheet was cut into strips (3 mm × 1 mm) using a cutter (SV-8, Roland DG). A PDMS ring (outer diameter: 8 mm, inner diameter: 6 mm) was prepared by using a biopsy punch (Kai Medical, Japan). A PET sheet (SFL-A4, AS ONE) was used to prepare a disk with a biopsy punch with a diameter of 8 mm. The disk was provided with two 2 mm holes for attaching a PDMS strip and a fiber sheet. The PDMS ring and the PET disk were attached. The fiber sheet was transferred onto the PET disk using an adhesive transfer tape (3M (registered trademark)). The PDMS strip was moved and attached to the PET disk with tweezers. A polyimide (PI) sheet (3-1966-06, AS ONE) was punched with a 1 mm biopsy punch to prepare a disk. This disk was attached to the PDMS strip to create a force sensor (a device for measuring contractile force) (Fig. 1). Before cell seeding, this device was treated with plasma for 2 minutes.

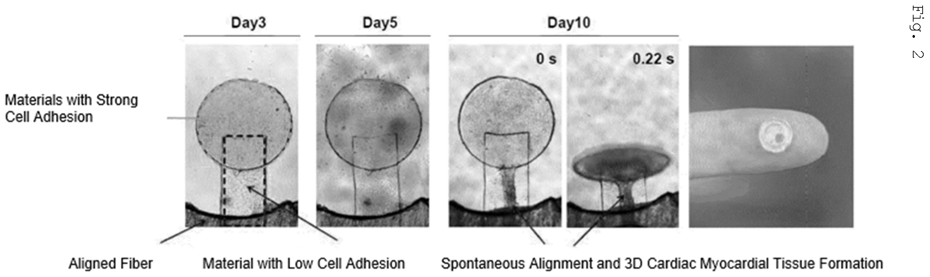

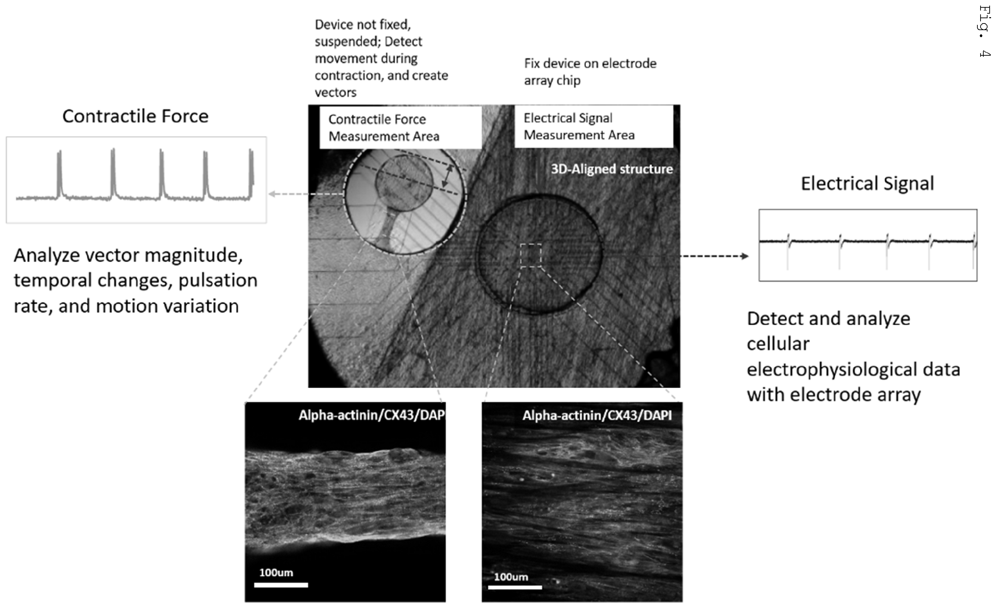

The present invention uses a three-dimensional, aligned fiber technology to construct a myocardial tissue with a three-dimensional, aligned structure. Additionally, an existing multi-electrode system for measuring electrical signals is applied, and a portion of the device is adhered to a multi-electrode chip to measure action potentials. Furthermore, during myocardial pulsation, with a portion of the device suspended, the amount of displacement of the outer portion of the round suspended portion is detected by image recognition technology, and the magnitude of the motion vector and the temporal change are recorded and reconverted into force by computing (Figs. 2 to 4).

The cells used for measurement cannot be assumed to be the same each time. As shown below, the use of the device produced in the present invention enabled the simultaneous measurement of electrical signals and changes in contractile force due to the addition of a drug by using the same cardiomyocytes under the same conditions.

### (3) Results of Measurement with Device

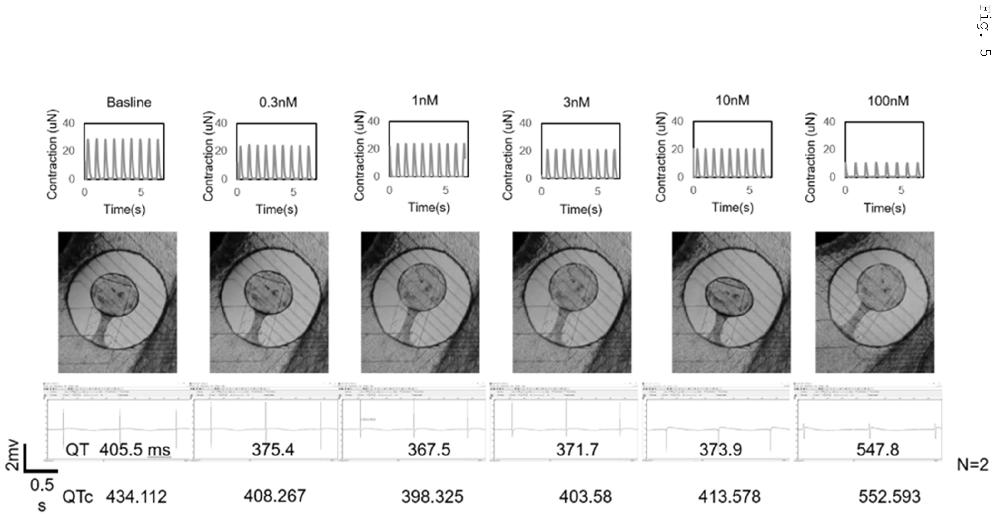

E4031 is an antiarrhythmic drug having the action of blocking HERG potassium channels. An increase in the concentration resulted in QT prolongation from 100 nM. With an increase in the concentration, the contractile force gradually weakened (Fig. 5).

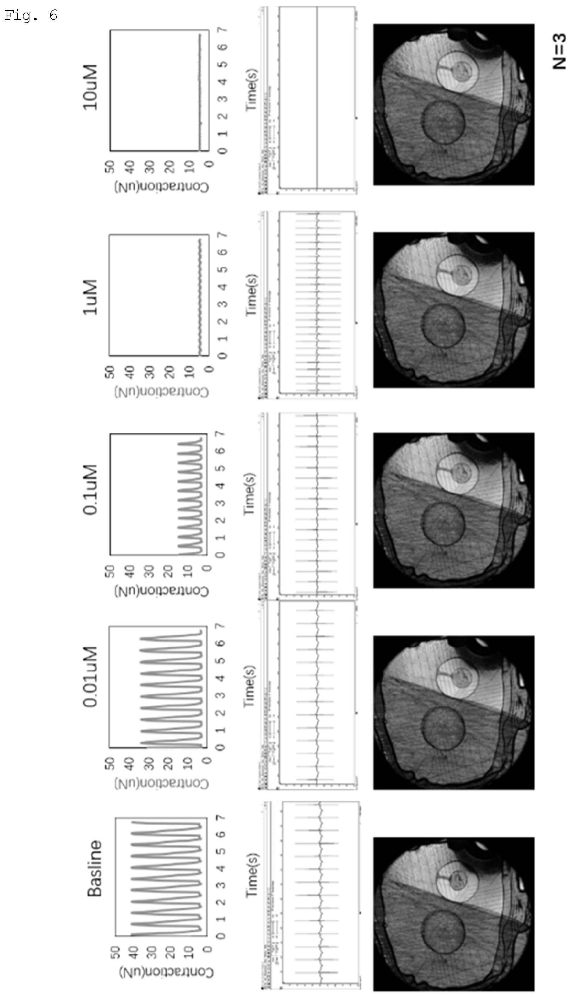

Verapamil is an antiarrhythmic drug having the action of blocking calcium channels. An increase in the concentration gradually accelerated the beating frequency. When the concentration was raised to 10 µM, the action potential ceased. The contractile force gradually weakened and became almost unmeasurable at a concentration of 1 µM (Fig. 6).

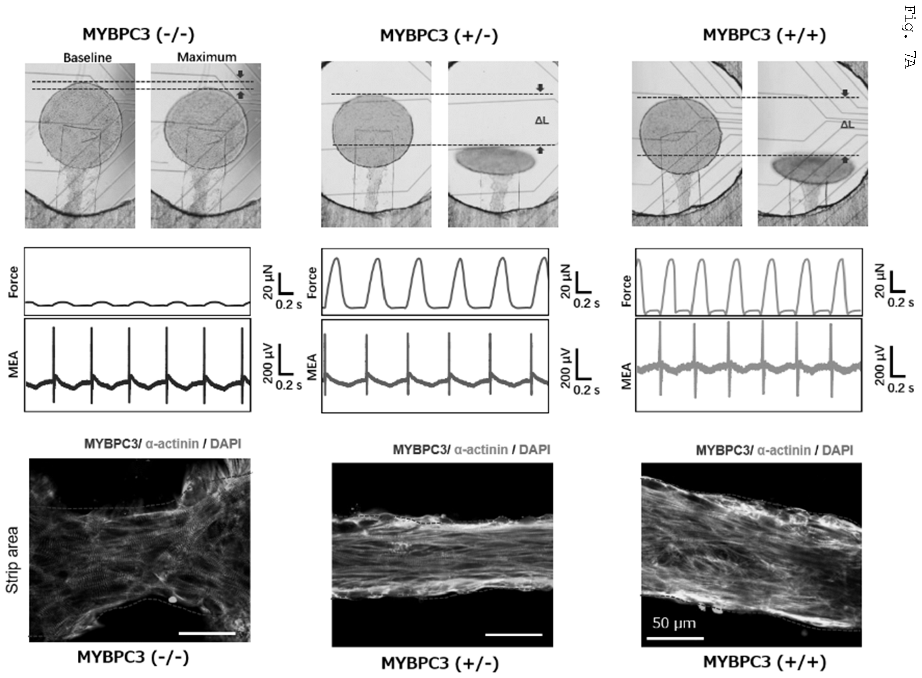

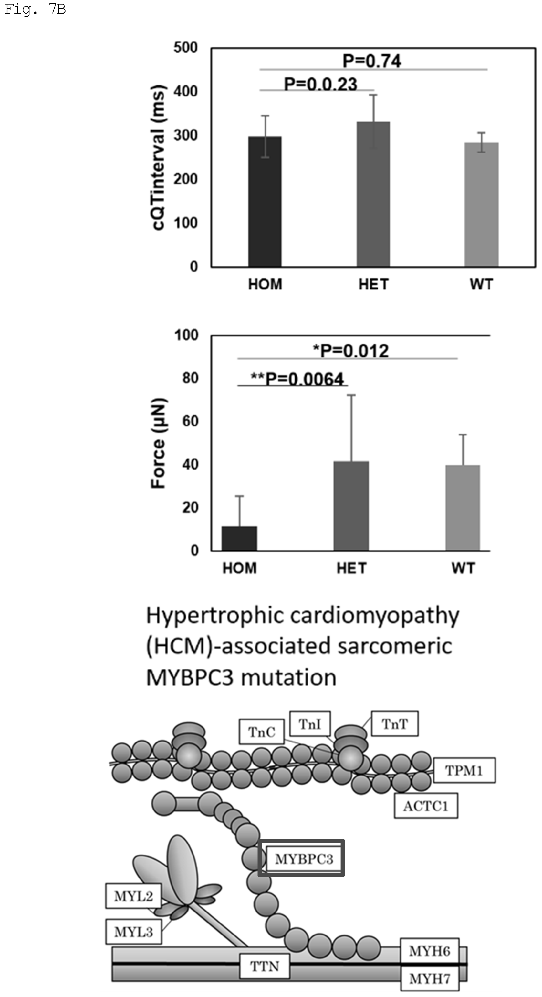

Furthermore, normal and diseased iPS cell-derived cardiomyocytes were used to evaluate drug response and to evaluate efficacy. The results indicated that no differences were observed in electrical signals and contractile force between cardiomyocytes heterozygous for the hypertrophic cardiomyopathy (HCM)-associated sarcomeric factor MYBPC3 (myosin-binding protein C) and normal cardiomyocytes. In contrast, in the homozygous mutant cardiomyocytes, no differences from normal or heterozygous cells were observed in electrical signals, but decreased contractile force was more clearly demonstrated by the technology of the present invention (Fig. 7).

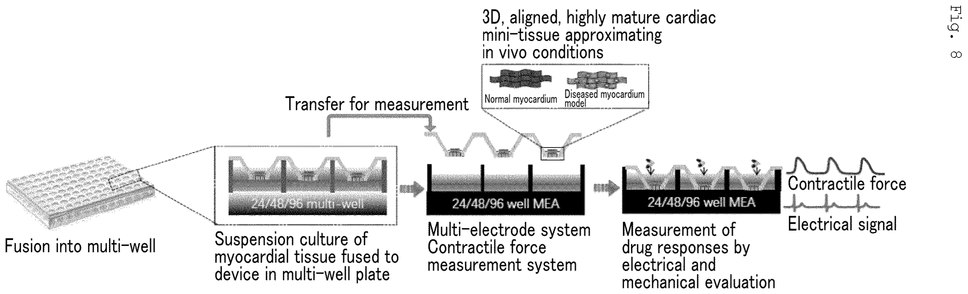

With conventional existing methods, samples have to be discarded once measured. However, the present system allows reuse of samples and of the evaluation system, and thus leads to cost reduction. The device can also be upgraded for 24, 48, or 96 wells according to the needs of easy handling, mass production, or high throughput in drug discovery (Fig. 8).

### (4) Measurement of Contact Angle of Various Materials with Water

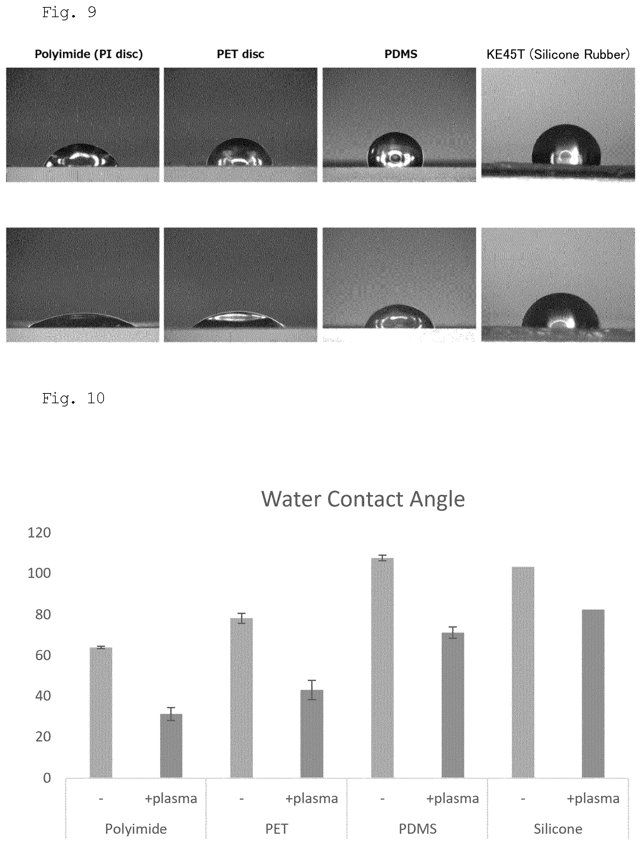

In addition to the various materials (polyimide, polyethylene terephthalate, and PDMS) used in the Examples above, silicone rubber was also separately prepared, and the contact angle with water was measured for each. The contact angle with water after plasma treatment was also measured in the same manner. Figs. 9 and 10 show the results.

## Claims

1. A device for measuring contractile force of myocardial tissue, comprising a contraction unit including a rod-shaped engineered myocardial tissue with a 3D structure in which cardiomyocytes are aligned in a long-axis direction,
wherein the device is for use in the measurement of contractile force of the contraction unit.

2. The device for measuring contractile force of myocardial tissue according to claim 1, wherein the engineered myocardial tissue is obtained by culturing cardiomyocytes on a surface having a contact angle with water of 95° or greater.

3. The device for measuring contractile force of myocardial tissue according to claim 1 or 2, further comprising a marker unit including a non-contractile myocardial tissue,
wherein the contraction unit is coupled to the marker unit so that the contractile force of the contraction unit is measurable according to the degree of displacement of the marker unit.

4. The device for measuring contractile force of myocardial tissue according to claim 1 or 2, further comprising an action potential measurement unit including an engineered myocardial tissue with a 3D structure in which cardiomyocytes are aligned,
wherein the action potential measurement unit is for use in the measurement of an action potential with multielectrodes.

5. A method for producing a rod-shaped engineered myocardial tissue with a 3D structure in which cardiomyocytes are aligned in a long-axis direction,
the method comprising the step of culturing cardiomyocytes on a surface having a contact angle with water of 95° or greater to obtain the engineered myocardial tissue.

6. The method according to claim 5, wherein
in the culturing step, a region having a surface with a contact angle with water of 95° or greater (A) is joined to two regions each having a surface with a contact angle with water of 85° or less (B), and
cardiomyocytes are cultured in the region (A) and the regions (B) to form the rod-shaped engineered myocardial tissue on a line connecting the two regions (B).
